# EUROPEAN PATENT APPLICATION

(11) **EP 2 213 248 A1**
(43) Date of publication of application: **04.08.2010**
(21) Application number: 10250138.4
(22) Date of filing: 28.01.2010
(51) Int. Cl.: A61B 17/06, A61B 17/00, A61B 17/02, A61B 17/34

(54) **Suture management apparatus for surgical portal apparatus including interlocking cap**

(30) Priority: 30.01.2009 US 148470 P; 05.01.2010 US 652381
(71) Applicant: Tyco Healthcare Group LP, North Haven, CT 06473 (US)
(72) Inventor: Bergeron, Richard, Wallingford, CT 06492 (US)
(74) Representative: Soames, Candida Jane

(57) **Abstract**

A surgical portal apparatus for use in a surgical procedure incorporating one or more sutures is provided. The surgical portal apparatus includes a portal member defining a longitudinal axis and having a longitudinal opening therethrough, and a suture management device operably connected to the portal member. The suture management device includes a first member and a second member mountable to the first member. The first member and the second member have cooperating surfaces adapted to selectively secure the at least one suture in a predetermined relation with respect to the portal member.

## Description

### CROSS REFERENCE TO RELATED APPLICATION

The present application claims the benefit of and priority to U.S. Provisional Application Serial No. 61/148,470 filed on January 30, 2009, the entire contents of which are incorporated herein by reference.

### BACKGROUND

### Technical Field

The present disclosure relates to a trocar and other surgical portal apparatus, and more particularly, relates to a suture management apparatus for surgical portal apparatus that includes an interlocking cap.

### Background of Related Art

Trocars and other surgical portal apparatus are known, as are myriad procedures that may be preformed using such assemblies. Many of the minimally invasive procedures performed through access assemblies necessitate or are simplified by the use of one or more sutures passing through the surgical portal apparatus. Sutures extending into a body cavity through a surgical portal apparatus may be used to, for example, temporarily retain tissue, manipulate tissue, anchor tissue or operate peripheral devices. In an attempt to reduce the number of incision sites required to complete a given surgical procedure, a single surgical portal apparatus may be used to pass one or more sutures into a body cavity, in addition to providing access for one or more devices. A single anchor device may have numerous suture ends that extend therefrom and through the surgical portal apparatus. The sutures extending through the surgical portal apparatus may become tangled as each is manipulated or as one or more instruments are inserted and withdrawn from the assembly. Also, a surgeon may confuse the suture ends during the course of a surgery. Tangling or confusion of the suture ends may unnecessarily complicate the procedure and increase time necessary to complete the procedure.

Therefore, it would be beneficial to have an apparatus for use with a surgical portal apparatus for managing sutures.

### SUMMARY

A surgical portal apparatus for use in a surgical procedure incorporating one or more sutures includes a portal member defining a longitudinal axis and having a longitudinal opening therethrough and a suture management device operably connected to the portal member. The suture management device includes a first member and a second member mountable to the first member. The first member and the second member have cooperating surfaces adapted to selectively secure the at least one suture in a predetermined relation with respect to the portal member. The first member may include at least one suture retaining slot. The second member includes at least one depending rib dimensioned for at least partial reception within the at least one slot of the first member. The at least one slot and the at least one rib may extend radially outwardly relative to the longitudinal axis.

In one embodiment, the first member may include a plurality of slots and the second member may include a plurality of ribs. The ribs are dimensioned and arranged to be partially received within respective slots to thereby releasably secure a plurality of sutures. The respective slots and recesses of the first and second members may be arranged in predetermined radial spaced relation.

The first member and the second member each define a longitudinal opening sized to receive a surgical object. The portal member may include a housing segment and an elongated segment extending from the housing segment. The suture management device is adapted to releasably engage the housing segment. Alternatively, the suture management device is integrally formed with the housing segment.

In another aspect, a suture management device includes an insert member configured to be operably connected to a portal member. The insert member includes a plurality of radially outward extending slots. A cap member includes a plurality of radially outward extending ribs configured to engage in the slots of the insert member, wherein engagement of the ribs in the slots securely retains at least one suture therein.

### BRIEF DESCRIPTION OF THE DRAWINGS

The accompanying drawings, which arc incorporated in and constitute a part of this specification, illustrate embodiments of the disclosure and, together with a general description of the disclosure given above, and the detailed description of the embodiment(s) given below, serve to explain the principles of the disclosure, wherein:
**FIG. 1** is a perspective side view of a surgical portal apparatus including a suture management apparatus according to an embodiment of the present disclosure;
**FIG. 2** is a perspective side view of the cap member of the suture management apparatus of FIG. 1;
**FIG. 3** is a perspective side view of the insert member of the suture management apparatus of FIG. 1; and
**FIG. 4** is a perspective side view of a surgical portal apparatus including a suture management system according to another embodiment of the present disclosure;

### DETAILED DESCRIPTION

The surgical portal apparatus herein disclosed may be configured for use in various surgical procedures, including laparoscopic, endoscopic, arthroscopic and orthopedic surgery. The access assembly provides passage between a subject's body cavity and the outside atmosphere and is capable of receiving surgical instruments of various sizes and configurations. An embodiment of the presently disclosed access assembly is configured to receive, for example, clip appliers, graspers, dissectors, retractors, staplers, laser probes, photographic devices, endoscopes and laparoscopes, tubes, and the like. Such instruments are collectively referred to herein as "instruments" or "instrumentation."

In addition to the instruments, the access assembly also allows the passage of one or more sutures therethrough, e.g., during an arthroscopic procedure. When several sutures are introduced into the subject's body through the access assembly, the sutures might tangle with each other or be confused by a surgeon. Suture tangle and/or confusion may, at the very least, inconvenience the clinicians conducting the surgical procedure. To minimize the possibility of sutures tangling with one another or a surgeon from confusing the sutures, the access assembly incorporates a suture retaining member for holding the one or more suture in place. The suture retaining member may also be used to maintain the one or more suture in a taut condition.

Referring now to the drawings wherein like reference numerals illustrate similar components throughout the several views, there is illustrated a suture management system in accordance with the principles of the present disclosure. In the following description, the term "proximal" refers to the portion of the access assembly that is closest to the clinician, whereas the term "distal" refers to the portion of the access assembly that is farthest from the clinician. As used herein, the term "subject" refers to a human patient or other animal. The term "clinician" refers to a physician, nurse or other care provider and may include support personnel.

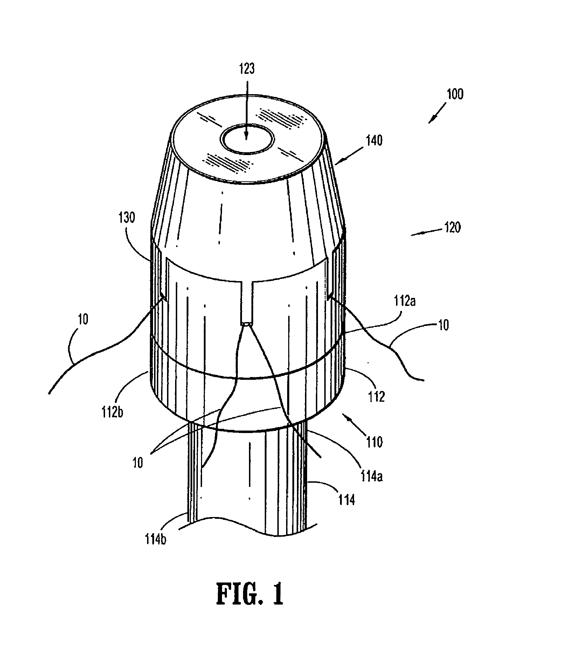

Referring initially to **FIG. 1****,** a suture management system according the present disclosure is shown as suture management system **100.** Suture management system **100** includes a surgical portal apparatus **110** and a suture management device **120.**

Surgical portal apparatus **110** includes a portal member **112** and a sleeve **114** extending distally from portal member **112.** Although the following discussion of suture management device **120** will be with respect to surgical portal apparatus **110,** the aspects of the present disclosure should not be read as limited to the embodiments herein disclosed. Suture management device **110** may be modified for use with any surgical portal apparatus.

Still referring to **FIG. 1****,** portal member **112** defines a substantially cylindrical housing having an open proximal end **112a** and a substantially open distal end **112h.** Open proximal end **112a** is configured to engage insert **120.** Distal end **112b** of portal member **112** may be integrally formed with sleeve **114.** Alternatively, portal member **112** may be configured for selectable engagement with sleeve **114.** Portal member **112** may be construction of plastic, polymer or other like material. Portal member **112** may be disposable, or in the alternative, reusable. Portal member **112** may be rigid, or alternatively, substantially flexible. Portal member **112** may include one or more seal members (not shown) having any seal arrangement for receiving a surgical object in a sealing manner. Portal member **112** may further include one or more anchors (not shown) or other suture securing means for securing one or more suture **"S"** extending through surgical portal apparatus **110.** Portal member **112** may further include an insufflation valve or port (not shown) configured to fill the body cavity of a patient with insufflation gas, saline or other suitable fluid.

Sleeve **114** is configured to be inserted through the skin into a body cavity with the aid of an obturator (not shown), or may instead, include a blade or piercing tip for penetrating through the skin and into a body cavity. Sleeve **114** forms a substantially tubular member having proximal and distal ends **114a, 114b** and defining a first longitudinal passage **113** extending therebetween. Sleeve **114** may be composed of plastic, metal, polymers or the like. Sleeve **114** may be disposable, or in the alternative, reusable. Sleeve **114** may be rigid, or alternatively, sleeve 114 may be flexible. Sleeve **114** may be open, or instead, may be configured to include one or more seal members (not shown) having any seal arrangement along the length thereof.

With reference now to **FIGS. 1-3****,** suture management device **120** includes a substantially cylindrical assembly configured to be received on proximal or housing end **112a** of portal member **112.** Suture management device **120** defines a longitudinal passage **123** configured for receipt of endoscopic, laparoscopic or other elongated instrument of various diameters. Suture management apparatus **120** includes an insert or first member **130** and a cap or second member **140** configured to engage insert member **130.** Although suture management apparatus **120** is preferably used with a surgical portal apparatus having one or more seal members for receiving an instrument in a sealed fashion, it is envisioned that suture management apparatus **120** may include one or more seal members (not shown).

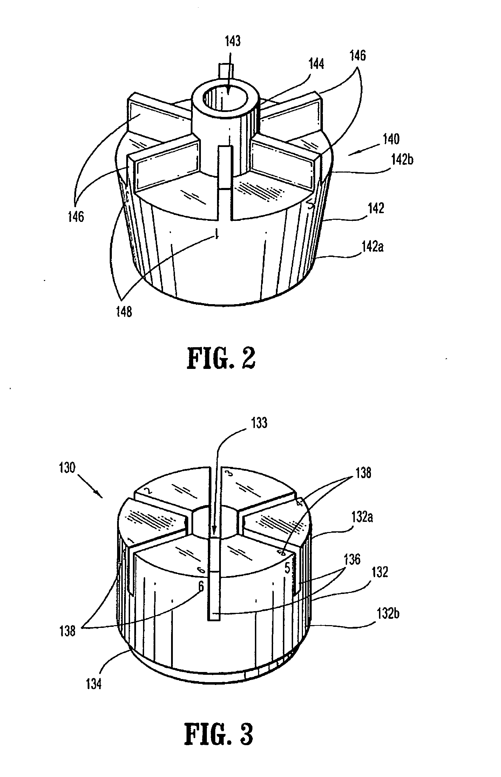

Referring now to **FIG. 3****,** insert member **130** defines a substantially cylindrical body **132** having proximal and distal ends **132a, 132b** and defining a longitudinal passage **133** extending therebetween. Distal end **132b** includes a flanged or recessed portion **134** configured to be received in proximal end **112a** of portal member **112.** As shown, insert member **130** is configured to frictionally engage proximal end **112a** of portal member **112;** however, it is envisioned that insert member **130** and portal member **112** may be mechanically coupled, i.e. threaded engagement. Although shown as being received within proximal end **112a** of portal member **112,** it is further envisioned that insert member **130** may be configured to be received about portal member **112.** Proximal end **132a** of insert member **130** includes a plurality of radial extending slots **136.** Slots **136** are spaced about proximal end **132a** and extend outwardly from longitudinal passage **133.** Insert member **130** may include any number of slots **136.** Slots **136** may be of varying widths. Each of slots **136** is configured to receive at least one suture **10 (****FIG. 1**). As shown, each of slots **136** may include markings **138** to assist in identifying sutures **10** received therein. Markings **138** may be letters, numbers, symbols, colors or other identifying feature. As will be discussed in further detail below, markings **138** may also assist in aligning cap member 140 with insert member **130.**

With reference now to **FIG. 2****,** cap member **140** defines a substantially frustro-conical body **142** having proximal and distal ends **142a, 142b** and defining a longitudinal passage **143** therebetween. Distal end **142** of cap member **140** includes a distal extension **144** extending therefrom. Distal extension **144** defines a distal end of longitudinal passage **143** and is configured to be received within longitudinal passage **133** of insert member **130.** Cap member **140** further includes a plurality of ribs **146** extending radially outward from distal extension **144.** Ribs **146** correspond in number and placement to slots **136** formed in proximal end **132a** of insert member **130.** Each of ribs **146** is configured to be received within a corresponding slot **136** of insert member **130.** Ribs **146** may be over-sized, include a coating (not shown), or otherwise be configured to frictionally engage slot **136** and/or sutures **10.** Distal end **142b** of cap member **140** may also include markings **148** corresponding to markings **138** on insert member **130.** Marking **148** may assist in aligning ribs **146** with slots **136** and/or, in lieu of markings **138** on insert member **130,** to identify sutures **10** within slots **136.**

Depending on the configuration of surgical portal apparatus **110** and/or the preference of the user, insert member **130** of suture management apparatus **120** may be attached to surgical portal apparatus **110** during manufacture, by a surgeon prior to insertion of surgical portal apparatus **110** into a body cavity or following insertion of the surgical portal apparatus **110** into the body cavity. Cap member **140** may be provided separate from insert member **130,** or alternatively, cap member **140** may be engaged with insert member **130.** As discussed above, sleeve **114** of surgical portal apparatus **110** may be of the self-piercing type or may instead be inserted with the assistance of an obturator (not shown). Prior to receipt of sutures **10 (****FIG. 1****)** within slots **136** of insert member **130,** surgical portal apparatus **110** functions as a traditional access assembly. A seal member (not shown) disposed within portal member **112,** sleeve **114** and/or suture management apparatus **120** may operate to receive instrument **"I"** in a sealed manner.

In operation, cap member **140** of suture management apparatus **120** is disengaged from insert member **130** in order to permit one or more sutures **10** to be received within slots **136** of insert member **130.** The sutures **10** may be used to manipulate tissue or in conjunction with, e.g. an orthopedic procedure to connect tissue. The sutures **10** may extend from the operative site through sleeve **114** of portal apparatus **110.** Once one or more sutures **10** are received within slots **136,** cap member **140** is engaged with insert member **130** such that ribs **146** engage slots **136** formed in insert member **130.** Sutures **10** are thus frictionally retained within slot **146** by ribs **136.** Alternatively, or additionally, distal extension **144** of cap member **140** frictionally engages sutures **10** against an inner surface of insert member **130.** Cap member **140** may be disengaged and reengaged from insert member **130** as necessary to add, remove and/or relocate sutures **10** within slots **136.** In using suture management system **100** during a surgical procedure, sutures **10** that would otherwise become tangled, misplaced and/or confused, are instead securely retained by suture management device **120** and maintained separate from instrument **"I".**

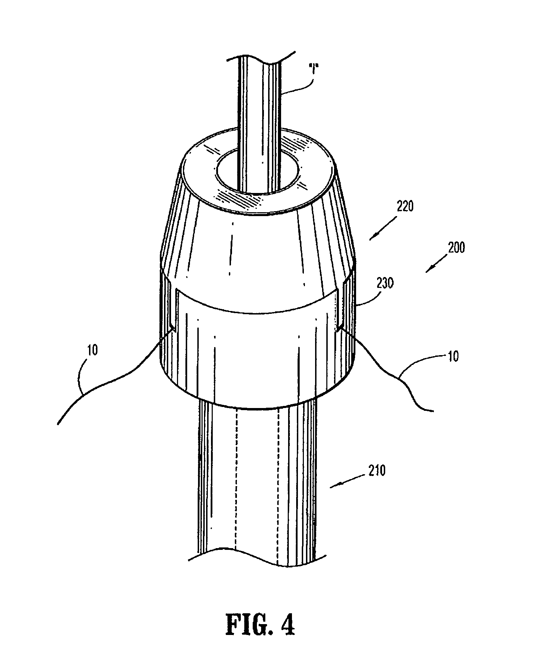

Turning now to **FIG. 4****,** an alternate embodiment of a suture management system is shown generally as suture management system **200.** Suture management system **200** is substantially similar in form and function to suture management system **100** described hereinabove. Suture management device **220** includes a suture management apparatus **220** and a surgical portal apparatus **210.** Suture management apparatus **220,** and insert member **230** in particular, is integrally formed with surgical portal apparatus **210.** In this manner, insert member **230** and surgical portal apparatus **210** cannot accidently become separated during a procedure.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

## Claims

1. A surgical portal apparatus for use in a surgical procedure incorporating one or more sutures, which comprises:
a portal member defining a longitudinal axis and having a longitudinal opening therethrough; and
a suture management device operably connected to the portal member, the suture management device including a first member and a second member mountable to the first member, the first member and the second member having cooperating surfaces adapted to selectively secure the at least one suture in a predetermined relation with respect to the portal member.

2. The surgical portal apparatus of claim 1, wherein the first member includes at least one suture retaining slot.

3. The surgical portal apparatus of claim 2, wherein the second member includes at least one depending rib dimensioned for at least partial reception within the at least one slot of the first member.

4. The surgical portal apparatus according to claim 3, wherein the at least one slot and the at least one rib extend radially outwardly relative to the longitudinal axis.

5. The surgical portal apparatus according to any preceding claim, wherein the first member includes a plurality of slots and the second member includes a plurality of ribs, the ribs dimensioned and arranged to be partially received within the respective slots to thereby releasably secure a plurality of sutures.

6. The surgical portal apparatus of claim 5, wherein the respective slots and recesses of the first and second members are arranged in predetermined radial spaced relation.

7. The surgical portal apparatus of claim 6, wherein the first member and the second member each define a longitudinal opening sized to receive a surgical object.

8. The surgical portal apparatus of any preceding claim, wherein the portal member includes a housing segment and an elongated segment extending from the housing segment, the suture management device being adapted to engage the housing segment.

9. The surgical portal apparatus of any preceding claim, wherein the suture management device is integrally formed with the portal member segment.

10. The surgical portal apparatus of any preceding claim, wherein at least one of the first member and the second member include markings for identifying the one or more sutures retained therein.

11. The surgical portal apparatus of any preceding claim, wherein at least one rib of the second member is dimensioned for reception within the at least one slot of the first member in frictional relation therewith.

12. A suture management device for use with a portal member, the device comprising:
an insert member configured to be operably connected to a portal member, the insert member including a plurality of radially outward extending slots; and
a cap member including a plurality of radially outward extending ribs configured to engage in the slots of the insert member, wherein engagement of the ribs in the slots securely retains at least one suture therein.
